# EUROPEAN PATENT APPLICATION

(11) **EP 4 830 625 A1**
(43) Date of publication of application: **28.10.2026**
(21) Application number: 26172728.3
(22) Date of filing: 16.04.2026
(51) Int. Cl.: A61B 5/145, A61B 5/00, A61M 5/172, A61M 5/142

(54) **GLUCAGON PATCH PUMP**

(30) Priority: 24.04.2025 US 202563794081 P
(71) Applicant: Willow Laboratories, Inc., Irvine, CA 92617 (US)
(72) Inventor: KIANI, Massi Joe E., Irvine, 92617 (US); VO, Hung The, Irvine, 92617 (US)
(74) Representative: Zacco Sweden AB

(57) **Abstract**

A glucagon patch pump designed for managing glucose levels in individuals, particularly those with diabetes. The glucagon patch pump includes a base that attaches to a patient's tissue site, a medication bladder for storing glucagon, a medication pump with a conduit for delivering the medication, and a cannula insertion device that deploys the cannula when glucose levels fall below a predetermined threshold. The system can automatically administer glucagon in response to low glucose levels, reducing the need for second-party intervention and minimizing the risk of prolonged hypoglycemia. The glucagon patch pump can be part of a comprehensive glucose management system that includes a glucose sensor and a processor to monitor and respond to glucose levels.

## Description

### FIELD OF THE DISCLOSURE

The general field of this disclosure is glucose sensing and disease management systems.

### BACKGROUND

Diabetes is a chronic disease that impacts many individuals, both adults and children. The management of diabetes may include the measurement of glucose within the interstitial space including blood and/or interstitial fluid of a patient and administration of insulin to the patient. A closed loop insulin administration system includes both a sensor to take glucose measurements from the interstitial space including blood and/or interstitial fluid of the patient and an insulin administration device which administers insulin to the patient based on the glucose measurements. Closed loop insulin administration systems allow individuals impacted by diabetes to go about daily life with much less worry about their insulin or glucose levels which can vastly improve a diabetic's quality of life.

### SUMMARY

In an aspect, a glucagon patch pump includes: a base configured to at least partially couple to a tissue site of a patient; a medication bladder coupled to the base and configured to store medication, the medication including glucagon; a medication pump coupled to the medication bladder, including a conduit, wherein the medication pump is configured to cause the medication to flow from the medication bladder through the conduit; and a cannula insertion device coupled to the medication pump via the conduit, wherein the cannula insertion device is configured to deploy the cannula when a user's glucose level drop below a predetermined threshold.

In some examples, the cannula insertion device includes a cannula, a spring, and a triggering component, wherein the cannula insertion device is configured to insert the cannula into the tissue site of the patient by actuation of the spring released by the triggering component. In some examples, the cannula can deploy if the user is in a safe pose when the glucose level drops below the predetermined threshold. The cannula may not deploy if the user is in an unsafe pose when the glucose level drops below the predetermined threshold.

In an aspect, a glucose management system including: the glucagon patch pump; a glucose sensor; a processor configured to receive measurements from the glucose sensor and determine a glucose level of the user.

In some examples, the system includes an insulin pump. In some aspects, the glucose sensor is a minimally invasive glucose sensor.

In an aspect, a method of treating hypoglycemia of a user, the method including: monitoring a user's blood glucose level using a glucose sensor; detecting when the user's blood glucose level drops below a predetermined threshold; deploying a cannula into a tissue site of the user in at least in part based on the detection of the blood glucose level dropping below the predetermined threshold; and administering medication through the cannula from a medication bladder via a medication pump, the medication including glucagon.

In some examples, the method includes issuing a notification to alert the user or a healthcare provider regarding the administration of glucagon or the user's blood glucose level. In some examples, the deploying includes actuating a triggering component to allow a pre-loaded spring to drive the cannula into the tissue site. In some examples, the method includes monitoring a pose of the user, where the deploying is based at least in part on the user being in a safe pose. In some examples, the glucose sensor is a minimally invasive glucose sensor. In some examples, the method includes adhering a glucagon patch pump to the user, the glucagon patch pump including the cannula, the medication bladder, and the medication pump. In some examples, the method includes connecting the glucagon patch pump to a communication network including the glucose sensor.

### BRIEF DESCRIPTION OF THE DRAWINGS

Embodiments of various inventive features will now be described with reference to the following drawings. Throughout the drawings, reference numbers may be reused to indicate correspondence between referenced elements. The drawings are provided to illustrate example embodiments described herein and are not intended to limit the scope of the disclosure.
FIG. 1 illustrates an example of a disease management environment.
FIG. 2 illustrates a block diagram of an example disease management system.
FIG. 3 illustrates a block diagram of an example disease management system that can administer glucagon in response to hypoglycemia.
FIG. 4 illustrates a method of using a glucagon patch pump for treating hypoglycemia.

### DETAILED DESCRIPTION

Although certain preferred embodiments and examples are disclosed below, inventive subject matter extends beyond the specifically disclosed embodiments to other alternative embodiments and/or uses and to modifications and equivalents thereof. Thus, the scope of the claims that may arise here from is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

Systems and methods described herein may be applicable to diabetic disease management or another individual condition that may be treated with an implant or other minimally or non-invasive device configured to monitor a patient state and deliver medication on an ongoing or temporary basis. While reference may be made to a specific disease, such as diabetes, systems and methods described herein may be applicable to other diseases and conditions. For example, systems and methods described herein may be applicable to Hypoglycemia, a condition in which an individual's blood sugar level is low, or Hyperglycemia, a condition in which an individual's blood sugar level is high.

While in some examples, systems and methods described herein may reference monitoring or sensing of a specific parameter or blood analyte, such as glucose, other physiological conditions, physiological states, physiological parameters, physiological markers, blood analytes, the like or a combination thereof may be monitored or determined in addition or in the alternative to glucose. Similarly, while in some examples, reference may be made to a specific type of sensor, such as a glucose sensor, other analyte or other sensors may additionally or alternatively be used. For example, a glucose sensor may be configured to additionally measure other analytes. Additionally or alternatively, while reference may be made to specific types of invasive or non-invasive sensors, such as an invasive glucose sensor, any type of invasive or non-invasive sensor may be used, such as a non-invasive analyte sensor.

Additionally or alternatively, while in some examples, systems and methods described herein may reference specific medication, such as insulin or glucagon, to be delivered to the individual, other medications, fluids, or treatments may be administered in addition or in the alternative to medications such as insulin or glucagon. Similarly, while in some examples, reference may be made a specific type of pump or other component associated with a medication or fluid, such as an insulin pump or glucagon pump, the components described herein may be used with any fluid or medication and may include any device or apparatus configured to administer the medication.

Additionally, while reference may be made to the use of a certain number or type of device, any combination and number of devices may be used, such as one, two, three, four or more redundant or different devices. In some examples, a single device may be used to manage aspects of an individual's health, such as a combined medication pump and analyte sensor. In some examples, redundant devices may be used to manage aspects of an individual's health, such as two combined medication pump and analyte sensor devices. In some examples, different devices may be used and in communication to manage different aspects of an individual's health or different aspects of disease management, such as a separate medication pump and analyte sensor. In some cases, administration can include medication injected intramuscularly, subcutaneously, or intravenously or received orally, rectally, or via intranasal spray.

Hypoglycemia refers to a low blood sugar (glucose) level and is a common complication of diabetes. The glucose level that defines hypoglycemia is variable; however, hypoglycemia is defined by the American Diabetes Association as a blood sugar level that is below 3.0 mmol/L (54 mg/dL). In general, symptoms of hypoglycemia do not usually manifest until levels have reached a low of between 2.8 to 3.0 mmol/L (50 to 54 mg/dL). Symptoms can include, but are not limited to, confusion, unconsciousness, tremors, sweating, palpitations, anxiety, depression, tearfulness and feeling of dread, restlessness, dilated pupils, a sensation of pins and needles or tingling, or other symptoms that generally require assistance from another person to treat. If left untreated, severe hypoglycemia can cause seizures, loss of consciousness, and in some cases, it can be fatal (for example, brain death, cardiac arrhythmias, etc.).

It has been discovered that insulin-dependent diabetics average two episodes of symptomatic hypoglycemia per week. Furthermore, research suggests that there may be hundreds of thousands of severe hypoglycemic events and tens of thousands hypoglycemic related deaths annually in the US. As many as 1 in 20 individuals with Type 1 diabetes will die from a hypoglycemic event. Furthermore, data suggests that approximately one-third of older adults with diabetes whom have experienced severe hypoglycemia died within three years of the incident. This evidence suggests that an episode of hypoglycemia may have long-lasting consequences. Accordingly, severe hypoglycemia is an under-recognized risk factor for death in those with diabetes, and the ongoing adoption of insulin regimens will likely continue to increase the number of hypoglycemic events observed annually.

Furthermore, some people may suffer from hypoglycemia unawareness: a complication of diabetes in which the person is unaware of the drop in blood sugar because it fails to trigger the secretion of epinephrine which generates the characteristic symptoms of hypoglycemia that serve to warn the individual of the dropping blood glucose. Hypoglycemia unawareness can result in prolonged exposure to hypoglycemia, which can result in severe side effects including, but not limited to, seizure, loss of consciousness, or brain damage. The development of hypoglycemia unawareness increases the difficulty in managing blood glucose, since the affected individual may not be aware of symptoms until it is too late to treat (for example, because the individual is unconscious) without assistance from another party. About one in five people with Type 1 diabetes and one in ten with insulin-treated Type 2 diabetes report experiencing hypoglycemia unawareness.

Several factors may increase the risk of hypoglycemia unawareness. For example, a drop in blood glucose levels while sleeping can increase the risk of hypoglycemia unawareness. Many people develop nocturnal hypoglycemia while they are asleep and are not awake to perceive the symptoms or treat episode. If this happens frequently, it can affect the individual's ability to detect hypoglycemia warning symptoms while awake. As another example, exercise may increase the risk of hypoglycemia unawareness. The likelihood of hypoglycemia is increased both during and after exercise, when the body's tissues are more sensitive to insulin. This effect can be delayed and occur up to 15 or more hours later, especially if the amount of exercise was unusually strenuous. As another example, having diabetes for a long time may increase the risk of hypoglycemia unawareness. One study found that nearly 50% of people who had used insulin for over twenty years had developed impaired hypoglycemia awareness, while only 20% of those who were on insulin for fewer than ten years experienced this problem. People who have had diabetes for longer are more likely to have been exposed to multiple episodes of hypoglycemia, which has been shown to contribute to hypoglycemia unawareness. As another example, cognitive awareness may increase the risk of hypoglycemia unawareness. As people age, they start to experience cognitive symptoms such as slowed thinking, confusion, and difficulty speaking at the same time as typical hypoglycemia symptoms like shakiness, hunger, or sweating. The cognitive symptoms can therefore interfere with recognition of hypoglycemia. At younger ages, people typically recognize hypoglycemia more frequently because they experience "autonomic" symptoms before "cognitive" symptoms. There is no exact age when this effect begins to take place, as the process of aging varies so widely. However, some studies have shown that hypoglycemia unawareness occurs more frequently in people above the age of 60. As another example, consuming alcohol may increase the risk of hypoglycemia unawareness. Consuming alcohol can, at least in the short term, lower an individual's ability to recognize typical symptoms and impair the liver's ability to release glucose when your blood sugar is too low. These effects typically last as long as it takes your body to process the alcohol.

In contrast to insulin which is a hormone that removes glucose from the blood, glucagon is a hormone that encourages release of glucose into the blood. Glucagon can be used as a tool to help during hypoglycemic events; however, glucagon injections are seldom used due at least in part to its side effects (for example, nausea, vomiting) and/or the typical requirement that a second party aid with the injection.

A glucagon injection generally includes a solution to be mixed in a several step process. The process can vary across embodiments but generally includes a second party (for example, friend, family member, or emergency responder) performing a series of tasks such as removing the syringe and glucagon vial from the case, removing the cap from the needle, removing the cap from the vial, injecting liquid contained in the syringe into the glucagon vial wherein the liquid and freeze-dried glucagon are mixed within the vial, drawing the solution into syringe, and injecting the solution into the individual's leg or abdomen. A liquid glucagon formulation also exists, but the process generally remains reliant on secondary operators.

In situations in which the individual is unconscious due to hypoglycemia, it can be important that the response (for example, the administration of glucagon) be immediate and effective. However, the difficulties inherent in the glucagon kit and process for administering the glucagon make such a response difficult for untrained friends and family. In general, the greater number of steps required to administer the glucagon, the greater opportunity for error and failure. Accordingly, there is need for alternative approaches for glucagon or other medication delivery beyond emergency kits such as those described above. This need is emphasized by a simulation study of injectable glucagon where 50% of caregivers and 80% of acquaintances failed to inject any glucagon and three participants delivered insulin instead of glucagon.

Disclosed is a disease management system configured to reliably, safely, and automatically administer medication to an individual experiencing a disease event, such as hypoglycemia, hyperglycemia, or certain severities of hypoglycemia or hyperglycemia. In this way, the disclosed disease management system can reduce an individual's risk of prolonged exposure to hypoglycemia/hyperglycemia, risk of death, risk of other complications, etc. In some cases, the disclosed disease management system eliminates or reduces a need for second party administration. In some cases, the disclosed disease management system reduces the mental burden of administering the medication, for example by reducing the number of steps to be performed by the individual or a second party. In some cases, as a safety measure, the disclosed disease management system can prevent medication administration while the individual in in certain poses (for example, on his back) and/or can instruct the individual to change poses to receive the medication. In some cases, the disclosed disease management system can include a "bypass" injection features, allowing the individual to initiate an administration. In some cases, the disclosed disease management system is a wearable, implantable, or minimally or non-invasive device.

A disease management system may not be ready to inject a medication due to, for example, warmup periods, stabilization periods, or end of life periods. Disclosed is a disease management system configured to allow a user-initiated injection to allow an individual to cause an injection of medication, for example if the individual knows they are in an emergency situation.

The present disclosure also describes an integrated system that can incorporate a dosage system and physiological measurement sensor into one unit. Various components of the integrated system are described in detail herein, such as various medication pumps and applicators that may be used to apply the unit to an individual.

### Environment Overview

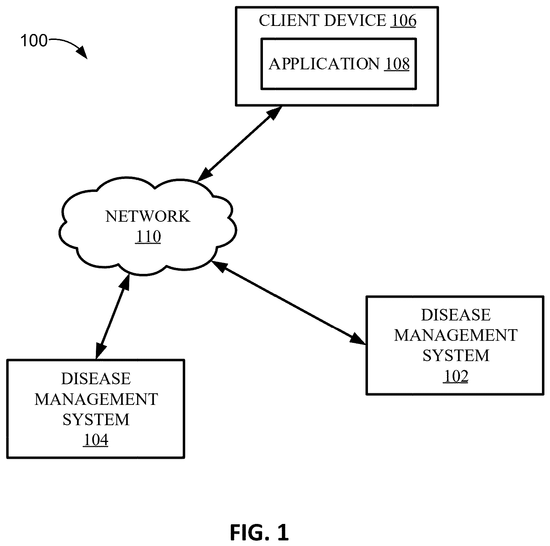

FIG. 1 illustrates an example of a disease management environment 100 that includes a disease management system 104, a disease management system 102, a client device 106, and a network 110. To simplify discussion and not to limit the present disclosure, Fig. 1 illustrates only one disease management system 102, disease management system 104, and client device 106, though multiple may be used. Furthermore, it will be understood that the environment 100 can include fewer, different, or additional devices or systems, as desired.

Any of the foregoing components or systems of the environment 100 may communicate, such as via the network 110. Although only one network 110 is illustrated, multiple distinct and/or distributed networks 110 may exist. The network 110 can include any type of communication network. For example, the network 110 can include one or more of a wide area network (WAN), a local area network (LAN), a cellular network (for example, LTE, HSPA, 3G, and other cellular technologies), an ad hoc network, a satellite network, a wired network, a wireless network, Bluetooth, and so forth. In some embodiments, the network 110 can include the Internet. In some cases, two or more of the components or systems of the environment 100 may be connected via a wired connection. In some cases, any one or any combination of the components or systems of the environment 100 may include an Ethernet adapter, cable modem, Wi-Fi adapter, cellular transceiver, baseband processor, Bluetooth or Bluetooth Low Energy (BLE) transceiver, or the like, or a combination thereof.

Any of the foregoing components or systems of the environment 100, such as any one or any combination of the disease management system 104, the disease management system 102, or the client device 106 may be implemented using individual computing devices, processors, distributed processing systems, servers, isolated execution environments (for example, virtual machines, containers, etc.), shared computing resources, or so on. Furthermore, any of the components or systems of the environment 100 may be combined and/or may include software, firmware, hardware, or any combination(s) of software, firmware, or hardware suitable for the purposes described. In some cases, a component or system of the environment 100 can be configured to communicate through another component or system of the environment 100. For example, in some cases, the client device 106 and the disease management system 104 can be configured to communicate through the disease management system 102.

The client device 106 may facilitate monitoring and/or management of one or more blood analytes, such a blood glucose. For example, as described herein, the client device 106 may provide an interface through which a user can interact with, control, or manage one or more of the disease management system 104 or the disease management system 102.

In some cases, the client device 106 device may implement an application 108. For instance, the client device 102 may represent any computing device capable of interacting with or running the application 108. The application 108 may facilitate monitoring and/or management of one or more blood analytes, such a blood glucose. The application 108 may include a web browser, a mobile application or "app," a background process that performs various operations with or without direct interaction from a user, or a "plug-in" or "extension" to another application, such as a web browser plug-in or extension. Although FIG. 1 illustrates the application 108 as being implemented on the client device 102, it will be understood that any of the components or systems of the environment 100 may host, execute, or interact with the application 108.

Examples of client devices 106 may include, without limitation, smart phones, tablet computers, handheld computers, wearable devices, laptop computers, desktop computers, servers, portable media players, gaming devices, and so forth. Furthermore, in some cases, the client device 106 may be integrated with one or more of the disease management system 104 or the disease management system 102.

The disease management system 102 can be used for continuous and/or spot check monitoring of one or more physiological parameters. Generally speaking, there can be at least two ways to obtain physiological parameter measurements. These include continuous monitoring and spot check measuring. A third approach is a hybrid of these two where an individual is monitored continuously for a short period of time to obtain a snapshot of physiological information. Continuous monitoring can involve taking measurements of an individual continuously or at least approximately continuously over an extended period of time. Spot check measurements, on the other hand, are typically performed as a single measurement at one point in time, instead of several measurements over a period of time as in continuous monitoring. A spot check measurement may be performed by placing a sensor on an individual, manually observing some characteristic of the individual, receiving a sample from an individual (for example, accepting and analyzing a test sample, such as via a test strip reader), etc. Like continuous measurements, spot check measurements (sometimes referred to herein simply as "spot checks") may be performed in a hospital or in any other setting, such as at an individual's home.

In some cases, the disease management system 102 may include a sensor that is attachable to an individual and configured to invasively, minimally invasively, and/or noninvasively obtain physiological signals. For example, the physiological signals may be obtained from any of a number of sensors including, but not limited to, optical sensors, piezoelectric sensors, electrical sensors, biomechanical sensors, or combinations of the same. For instance, optical sensors may provide parameters such as oxygen saturation or SpO₂, pulse rate, pleth variability index (PVI), perfusion index (PI), total hemoglobin or SpHb, methemoglobin or SpMet, carboxyhemoglobin or SpCO, blood glucose concentration or level, insulin concentration or level, glucagon concentration or level, among others. A piezoelectric sensor may be used to calculate parameters such as respiratory rate and pulse rate. Electrical sensors can be used to calculate parameters such as respiratory rate, heart rate, and other ECG-related parameters obtained from the electrocardiogram. Biomechanical sensors, such as bioimpedance sensors, can be also used to capture parameters like respiratory rate. In some cases, the sensor includes a pose sensor, as described herein. Invasive or minimally invasive sensors may provide parameters pertaining to blood analyte concentration, for instance blood glucose concentration or level, insulin concentration or level, glucagon concentration or level, among others. Such invasive or minimally invasive sensors may

In some cases, the disease management system 102 may be configured to obtain a specimen sample (for example, blood, urine, or other bodily fluid) from a user and can determine one or more physiological parameter measurements based at least in part on the specimen sample. The disease management system 102 can obtain the sample in a variety of ways. For example, the disease management system 102 may include a reception region in which the disease management system 102 can receive a sample via a test strip, swap, container, droplet, etc.

The one or more physiological parameters monitored or determined by the disease management system 102 can vary across examples. In some examples, the one or more physiological parameters can include, but are not limited to, blood glucose concentration or level, insulin concentration or level, glucagon concentration or level, oxygen saturation or SpO₂, pulse rate, pleth variability index (PVI), perfusion index (PI), total hemoglobin or SpHb, methemoglobin or SpMet, carboxyhemoglobin or SpCO, respiratory rate, heart rate, among others. The physiological parameter can include temperature of an individual.

In some examples, the disease management system 102 is configured to obtain measurements associated with blood glucose. In some such cases, the disease management system 102 may include a blood glucose monitor (for example, continuous glucose monitor (or CGM)), a test strip reader, a test strip, a lancet, or a display to display results, etc. For instance, the disease management system 102 can include a blood glucose monitor or sensor similar to that described in U.S. Pat. Pub Nos. 2012/0226117, entitled "Handheld Processing Device Including Medical Applications for Minimally and Noninvasive glucose Measurements," filed November 20, 2011, published September 6, 2012, or 2012/0296178, entitled "Personal Health Device," filed May 16, 2012, published November 22, 2012, each of which is hereby incorporated in its entirety by reference herein. A blood glucose monitor may be an invasive, minimally invasive, or noninvasive monitor.

The disease management system 104 can be used to facilitate administration of a medication to an individual, such as hormones (for example, glucagon, insulin, etc.) or other substances. In some cases, the disease management system 104 may be configured to provide an alert to the individual or a caregiver as to when to perform an administration (for example, intravenously, intramuscularly, subcutaneously in a kit that contains a vial and a syringe). In some cases, the disease management system 104 may be configured to perform the administration. For example, the disease management system 104 may be configured to couple to an individual and perform administrations with or without intervention from a user (e.g., automatically). In some cases, the disease management system 104 can be implemented as a wearable device that a user can wear directly on their body. As another example, the disease management system 104 can be implemented as an implantable device. In some cases, the disease management system 104, or the combination of the disease management system 104 and the disease management system 102, can provide a closed loop medication administration system, which can allow individuals impacted by particular ailments (for example, diabetes) to go about daily life without having to worry about their insulin or glucose levels, which can vastly improve a diabetic's quality of life.

In some cases, the particular medication (for example, glucagon, insulin, etc.) and/or amount administered is based at least in part on the one or more physiological parameter measurements obtained by the disease management system 102. For example, in some cases, the disease management system 104 can administer medications to the individual in order to regulate or affect the one or more physiological parameters measured by the disease management system 102. Consider a scenario in which the disease management system 102 monitors blood glucose. In some such cases, the disease management system 104 may facilitate the administration of insulin or glucagon to the individual based on the levels of blood glucose in the individual. For instance, based at least in part on a determination that a user's blood glucose level does not satisfy a blood glucose threshold (for example, indicating that blood sugar levels are too low), the disease management system 104 can cause or cause or perform administration (for example, of glucagon) or end an administration (for example, of insulin). As another example, based at least in part on a determination that a user's blood glucose level satisfies a second blood glucose threshold (for example, indicating that blood sugar levels are too high), the disease management system 104 can cause or perform administration (for example, of insulin) or end an administration (for example, of glucagon).

In some examples, the disease management system 104 may be utilized as an emergency or backup administration system. For example, the disease management system 104 may trigger an administration during a life threatening situation, such as when a user's blood sugar levels are too high or too low. Furthermore, in some cases, the disease management system 104 may cause an audible, visual, or other indication to alert the user that a life threatening situation is occurring, to alert the user that administration is or will be occurring, to provide instructions to the user.

In some cases, the disease management system 104 is configured to administer medication. In some cases, the disease management system 104 can deliver the medication within a threshold period of time. In some cases, the disease management system 104 can eliminate or reduce the need for second party assistance. In some cases, the disease management system 104 requires little or no interaction from a user in order to administer the medication to the individual. In some cases, the disease management system 104 is easy to apply and remove. In some cases, the disease management system 104 is configured to stay on the body. In some cases, the disease management system 104 is small in size so as the minimize body real estate requirements. In some cases, the disease management system 104 is discreet. In some cases, the disease management system 104 has remote administration capability, for example, via the client device 106. In some cases, the disease management system 104 has longer device wear times. In some cases, the disease management system 104 is water resistant or water proof. In some cases, the disease management system 104 is not greatly affected by altitude, humidity, or temperature.

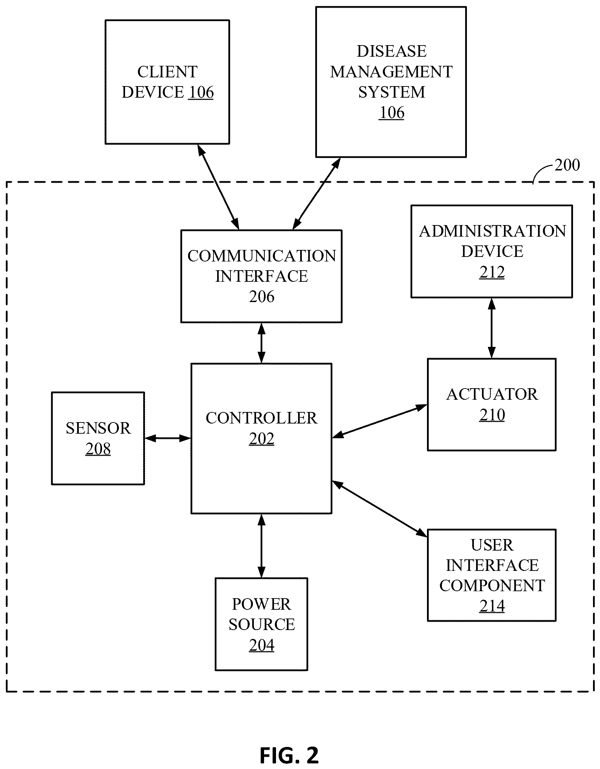

FIG. 2 illustrates a block diagram of an example disease management system 200, which may be an example of the disease management system 104 of FIG. 1. As shown, the disease management system 200 can include a controller 202, a power source 204, a communication interface 206, a sensor 208, an actuator 210, an administration device 212, or a user interface component 214. However, it will be understood that the disease management system 200 can include fewer, different, or additional devices or systems, as desired. Although the disease management system 200 is shown as separate from the disease management system 102 and client device 106, in some cases, the disease management system 200 can include or be integrated with the disease management system 102 or client device 106. Additionally, the components of the disease management system 200 (e.g., the controller 202, the power source 204, the communication interface 206, the sensor 208, the actuator 210, the administration device 212, or the user interface component 214) may be integrated into one or more devices. In some examples, the actuator 210 and the administration device 212 can be integrated to in a medication pump device.

The power source 204 can provide power to the disease management system 200. For example, the power can include a power supply (for example, one or more batteries, a wall outlet connector, etc.) and electronics to drive the controller 202, communication interface 206 (for example, transceiver), sensor 208, actuator 210, administration device 212, or user interface component 214.

The communication interface 206 can facilitate wired or wireless communication with one or more other systems or devices, such as the disease management system 102, the client device 106, the application 108, the network 110, etc. For example, the communication interface 206 can include a transceiver that includes an antenna. The communication interface 206 can be configured for any of a variety of applications, such as satellite technology (e.g. GPS), Bluetooth, BLE, Wi-Fi, near-field communication (NFC), mobile networks (e.g. 3G and 4G), or any combination thereof.

In some cases, the disease management system 200 can be configured to provide an alter based on the identification of a disease events. The alert can include an audible, visual, vibrotactile or other indication. For example, in some cases, the alert may include turning on the GPS, activating location services, enabling a 3G/4G/5G system, or the like in response to an identification of a disease event. This alert would enable localization of the person in emergency.

The sensor 208 can include a pose sensor configured to obtain pose data usable to determine a pose of an individual. The term "pose" is a broad term encompassing its plain and ordinary meaning and may refer to, without limitation, position and/or orientation or any other appropriate location information. The pose sensor can include, but is not limited to, any one or any combination of an accelerometer, gyroscopes, orientation sensor, gravity sensor, GPS or other location-related device, etc. The sensor 208 can include a physiological sensor, as described herein. For example, the disease management system 200 can receive physiological data and determine measurements of one or more physiological parameters, similar to the disease management system 200, as described herein. The sensor information may be processed and communicated to the controller 202. Similarly, the controller 202 may receive physiological data, such as from the disease management system 102.

The combination of the actuator 210 and the administration device 212 can deliver medication. For example, in some cases, the medication can include glucagon, a hormone that raises blood glucose levels. Although implementations can vary across embodiments, in some cases, the actuator 210 can be part of a medication pump that can administer medication to an individual with or without input from the individual. The medication pump can be, for example, a patch pump.

The user interface component 214 can include one or more buttons, switches, speakers, microphones, displays, or any combination thereof. For example, the user interface component 214 can include a speaker for alarms, such as voice directed alarms. As another example, the user interface component 214 can include a screen for a visual display or one or more status light indicators. In some cases, the user interface component 214 can be part of the client device 106.

The controller 202 can process data, such as sensor data from the sensor 208, or data received from the disease management system 102 or the client device 106. In some cases, controller 202 is capable of executing an application or providing a graphical user interface, which may be interacted with by a user to adjust settings, administer glucose, pair with other devices, calibrate, or the like.

Continuous glucose monitors (CGMs) measure the body's blood glucose (blood sugar) levels in real-time by sensing the glucose present in tissue fluid. CGMs are often configured to trigger an alarm when the blood sugar is too low (Hypoglycemia) or too high (Hyperglycemia). CGMs are often configured to trigger an alarm when the patient's blood sugar is too low or too high. These alarms have proven valuable in efforts to reduce the likelihood of conditions such as diabetic comas or the "dead-in-bed" syndrome. However, some manufacturers require alarms that cannot be silenced. Furthermore, many manufacturers require sensitive alarm settings, which tend to produce numerous false-alarms. The false-alarm, coupled with the inability to silence the alarms, can cause sleep disruption or insomnia.

Traditionally, the CGM alarm settings can be associated with a "time to react." The time to react can be related to an expected need to perform a sequence of events (for example, wake up, take a validation measurement while possibly impaired, administer a lifesaving emergency glucagon syringe) before losing consciousness, which occurs around 20 mg/dL. Table 1 illustrates various blood sugar thresholds (in mg/dL) and the expected amount of time (in min) to react at those thresholds. As shown, when assuming a maximum possible physiologic rate of change of glucose of -3 mg/dL, an individual with a blood sugar of 70 mg/dL has approximately 16 minutes to mitigate her condition before she becomes unconscious. Further, an individual with a blood sugar of 60, 50, and 40 mg/dL has approximately 13, 10, and 7 minutes, respectively, to mitigate her condition before she becomes unconscious. The traditional minimum non-silenceable alarm is set to 50 mg/dL.

**Table 1: Blood sugar levels and expected time to react before unconsciousness.**

| **Threshold [mg/dL]** | **Time to react [min]** |
|---|---|
| 70 | 16 |
| 60 | 13 |
| 50 | 10 |
| 40 | 7 |

Disclosed herein is a disease management system that can address at least the aforementioned or other concerns. For example, the disease management system can address safety concerns of administering glucagon. For example, the disease management system can deploy a glucagon cannula only when glucose levels drop too low. This may help to prevent premature administration of glucagon or reduce pain experienced by a user. In addition or alternatively, the disease management system can allow the minimum non-silenceable alarm to be lowered, thereby reducing the number of false alarms and reducing the instances of sleep disruption or insomnia. Additionally or alternatively, by monitoring an orientation of the individual, the disease management system can prevent administration of glucagon to an individual while the individual is lying on his back, thereby reducing the risk of choking given that glucagon administration tends to cause vomiting.

Continued false alarms are expected to continue to make persons with diabetes lifestyle difficult. This is due to the expectation that today's CGMs will not produce accuracy beyond 8 % MARD (due to lag-time issues) and that control algorithms will take on more function for the user autonomously. Some manufacturers require alarms that cannot be silenced and produce general insomnia as a result. One way of addressing this issue is by mitigating the issue of hypoglycemia. Alarms are set very high because a subject can only manage their state if they are conscious. If a device were to have a failsafe measure to manage the hypoglycemic state, one could argue the limit could be brought lower and remove a significant number of false alarms.

Regarding the conventional sensitive alarm settings, the disease management system provides a device to manage the hypoglycemic state, which can allow the minimum non-silenceable alarm to be lowered. For example, in the event of a life-threatening situation relating to blood sugar, the disclosed disease management system can automatically administer glucagon to the individual. Table 2 illustrates various blood sugar thresholds (in mg/dL) and the expected amount of time (in min) to react at those thresholds. As shown, the threshold when using the disclosed disease management system can be safely lowered to 40 mg/dL, thereby significantly reduce false-alarms.

**Table 2: Blood sugar levels and expected time to react before unconsciousness.**

| Threshold without emergency glucose [mg/dL] | Time to react [min] | Threshold with emergency glucagon [mg/dL] |
|---|---|---|
| 70 | 16 | 50 |
| 60 | 13 | 45 |
| 50 | 10 | 40 |
| 40 | 7 | 30 |

The administration of certain medications (for example, glucagon) can often induce vomiting. Accordingly, prior to administering the medication to an individual, it can be prudent to ensure that the individual is not in a pose (for example, lying on his back) that might lend to choking, should vomiting ensue.

To address these or other concerns, disclosed herein is a disease management system configured to determine a pose of an individual. Based on the pose, the disease management system can determine whether to administer the medication. In some cases, if the disease management system determines that the pose corresponds to any of a groups of poses that might lend to choking, the disease management system can refrain from administering the medication for a period of time and/or activate an alarm or instruction to attempt to cause the individual to change her pose or move to a particular pose so that the medication can be more safely administered. In this way, the disease management system reduces the likelihood that an individual will choke, should the medication cause the individual to vomit.

### Overview of Glucagon Patch Pump

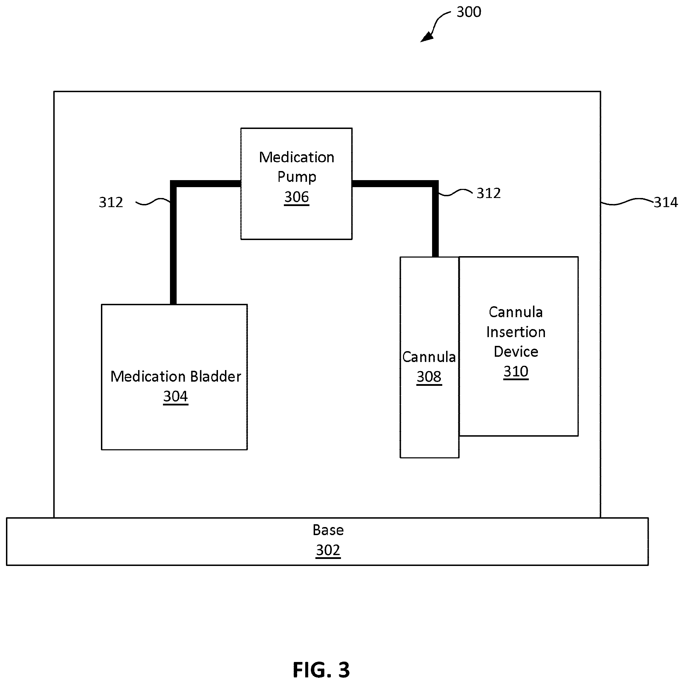

FIG. 3 illustrates a block diagram of an example disease management system 300, which may be an example of the disease management system 104 of FIG. 1. The disease management system 300 can be, for example, a glucagon patch pump. The disease management system 300 can include a base 302, a medication bladder 304, a medication pump 306, a cannula 308, a cannula insertion device 310, a conduit 312, and a housing 314. The disease management system 300 can include one or more processors that can control the medication pump 306 and the cannula insertion device 310. The one or more processors may be capable of receiving a signal indicative of a glucose level of the user.

The base 302 may include an adhesive layer that can secure the disease management system 300 to skin of a user. The medication bladder 304, the medication pump 306, and/or the cannula insertion device 310 may be secured to the base 302 directly or indirectly. In some examples, the medication bladder 304, the medication pump 306, and/or the cannula insertion device 310 are secured to the housing 314, which is secured to the base 302.

The cannula insertion device 310 can actuate the cannula 308 to deploy the cannula 308 into skin of the user. The cannula insertion device 310 can be controlled (e.g., by one or more processors of the disease management system 300). The cannula insertion device 310 can actuate the cannula 308 in response to the user's blood glucose levels falling below a predetermined threshold. The predetermined threshold may be set by the user, a health practitioner, or guidance standards. In some examples, the cannula insertion device 310 may include a spring (e.g., a pre-loaded spring) and a triggering component. The cannula insertion device 310 can actuate the spring by releasing the triggering component.

The medication pump 306 can be coupled to the medication bladder 304 and/or the cannula 308 via the conduit 312. The medication pump 306 can cause medication stored in the medication bladder 304 to flow from the medication bladder 304 through the conduit 312 to the cannula 308. In some examples, the medication pump 306 is capable of delivering small, precise dosages of the medication. In some examples, the medication stored in the medication bladder 304 can include glucagon. In some examples, the medication pump 306 can only activate once the cannula 308 has been deployed to the user.

### Method of Using Glucagon Patch Pump

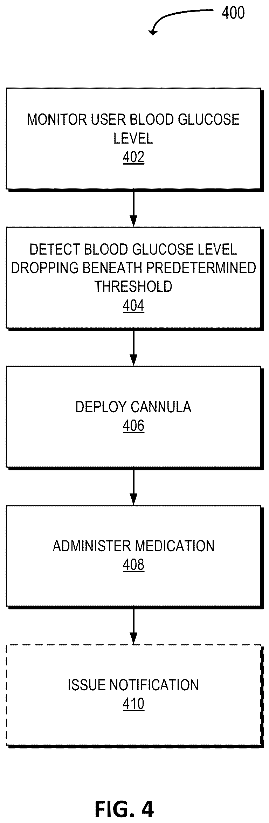

FIG. 4 illustrates a method 400 of using a glucagon patch pump, for example the disease management system 300 of FIG. 3. The glucagon patch pump can be worn by a user. In some examples, the patch pump can be adhesively secured to a user before the start of the method 400.

At step 402, monitor the user's blood glucose level. In some examples, step 402 can be carried out using, for example, a separate disease management system capable of measuring the user's blood glucose levels. Such a disease management system may include an invasive, minimally invasive, or noninvasive blood glucose sensor.

At step 404, detect glucose level dropping beneath a predetermined threshold. Detection of the blood glucose level dropping beneath the threshold can be based at least in part on the blood glucose level as measured by the blood glucose sensor. The predetermined threshold may be set by the user, a healthcare practitioner, or guidance standards.

At step 406, deploy the cannula. In some examples, a cannula insertion device can deploy the cannula by inserting the cannula into skin of the user. The cannula insertion device can release a triggering component to allow a preloaded spring to drive the cannula into the user's skin.

At step 408, administer medication. A pump of the glucagon patch pump can cause medication to flow from a medication bladder of the glucagon patch pump through a conduit to the cannula, where it is injected into the user. The medication can include glucagon.

At step 410, optionally issue a notification. In some examples, the glucagon patch pump or other device in the disease management environment can cause an alarm, an audible notification, a visual notification, or the like to the user or a healthcare provider. In some examples, the notification can indicate that the user's blood glucose level fell below the predetermined threshold. In some examples, the notification can indicate that the cannula was deployed and/or that medication was administered. In some examples, the notification can indicate that the user's blood glucose level increased over the predetermined threshold in response to the administration of the medication.

### Terminology and Additional Considerations

All of the methods and jobs described herein may be performed and fully automated by a computer system. The computer system may, in some cases, include multiple distinct computers or computing devices (e.g., physical servers, workstations, storage arrays, cloud computing resources, etc.) that communicate and interoperate over a network to perform the described functions. Each such computing device typically includes a processor (or multiple processors) that executes program instructions or modules stored in a memory or other non-transitory computer-readable storage medium or device (e.g., solid state storage devices, disk drives, etc.). The various functions disclosed herein may be embodied in such program instructions, or may be implemented in application-specific circuitry (e.g., ASICs or FPGAs) of the computer system. Where the computer system includes multiple computing devices, these devices may, but need not, be co-located. The results of the disclosed methods and jobs may be persistently stored by transforming physical storage devices, such as solid-state memory chips or magnetic disks, into a different state. In some embodiments, the computer system may be a cloud-based computing system whose processing resources are shared by multiple distinct business entities or other users.

Depending on the embodiment, certain acts, events, or functions of any of the processes or algorithms described herein can be performed in a different sequence, can be added, merged, or left out altogether (e.g., not all described operations or events are necessary for the practice of the algorithm). Moreover, in certain embodiments, operations or events can be performed concurrently, e.g., through multi-threaded processing, interrupt processing, or multiple processors or processor cores or on other parallel architectures, rather than sequentially.

The various illustrative logical blocks, modules, routines, and algorithm steps described in connection with the embodiments disclosed herein can be implemented as electronic hardware, or combinations of electronic hardware and computer software. To clearly illustrate this interchangeability, various illustrative components, blocks, modules, and steps have been described above generally in terms of their functionality. Whether such functionality is implemented as hardware, or as software that runs on hardware, depends upon the particular application and design conditions imposed on the overall system. The described functionality can be implemented in varying ways for each particular application, but such implementation decisions should not be interpreted as causing a departure from the scope of the disclosure.

Moreover, the various illustrative logical blocks and modules described in connection with the embodiments disclosed herein can be implemented or performed by a machine, such as a processor device, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor device can be a microprocessor, but in the alternative, the processor device can be a controller, microcontroller, or state machine, combinations of the same, or the like. A processor device can include electrical circuitry configured to process computer-executable instructions. In another embodiment, a processor device includes an FPGA or other programmable device that performs logic operations without processing computer-executable instructions. A processor device can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor device may also include primarily analog components. For example, some or all of the algorithms described herein may be implemented in analog circuitry or mixed analog and digital circuitry. A computing environment can include any type of computer system, including, but not limited to, a computer system based on a microprocessor, a mainframe computer, a digital signal processor, a portable computing device, a device controller, or a computational engine within an appliance, to name a few.

The elements of a method (including computer-implemented method), process, routine, or algorithm described in connection with the embodiments disclosed herein can be embodied directly in hardware, in a software module executed by a processor device, or in a combination of the two. A software module can reside in RAM memory, flash memory, ROM memory, EPROM memory, EEPROM memory, registers, hard disk, a removable disk, a CD-ROM, or any other form of a non-transitory computer-readable storage medium. An exemplary storage medium can be coupled to the processor device such that the processor device can read information from, and write information to, the storage medium. In the alternative, the storage medium can be integral to the processor device. The processor device and the storage medium can reside in an ASIC. The ASIC can reside in a user terminal. In the alternative, the processor device and the storage medium can reside as discrete components in a user terminal.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Disjunctive language such as the phrase "at least one of X, Y, Z," unless specifically stated otherwise, is otherwise understood with the context as used in general to present that an item, term, etc., may be either X, Y, or Z, or any combination thereof (e.g., X, Y, and/or Z). Thus, such disjunctive language is not generally intended to, and should not, imply that certain embodiments require at least one of X, at least one of Y, or at least one of Z to each be present.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations. For example, "a processor configured to carry out recitations A, B and C" can include a first processor configured to carry out recitation A working in conjunction with a second processor configured to carry out recitations B and C. Unless otherwise explicitly stated, the terms "set" and "collection" should generally be interpreted to include one or more described items throughout this application. Accordingly, phrases such as "a set of devices configured to" or "a collection of devices configured to" are intended to include one or more recited devices. Such one or more recited devices can also be collectively configured to carry out the stated recitations. For example, "a set of servers configured to carry out recitations A, B and C" can include a first server configured to carry out recitation A working in conjunction with a second server configured to carry out recitations B and C.

While the above detailed description has shown, described, and pointed out novel features as applied to various embodiments, it can be understood that various omissions, substitutions, and changes in the form and details of the devices or algorithms illustrated can be made without departing from the spirit of the disclosure. As can be recognized, certain embodiments described herein can be embodied within a form that does not provide all of the features and benefits set forth herein, as some features can be used or practiced separately from others. The scope of certain embodiments disclosed herein is indicated by the appended claims rather than by the foregoing description. All changes which come within the meaning and range of equivalency of the claims are to be embraced within their scope.

## Claims

1. A glucagon patch pump comprising:
a base configured to at least partially couple to a tissue site of a patient;
a medication bladder coupled to the base and configured to store medication, the medication comprising glucagon;
a medication pump coupled to the medication bladder, comprising a conduit, wherein the medication pump is configured to cause the medication to flow from the medication bladder through the conduit; and
a cannula insertion device coupled to the medication pump via the conduit, wherein the cannula insertion device is configured to deploy the cannula when a user's glucose level drop below a predetermined threshold.

2. The glucagon patch pump of claim 1, wherein the cannula insertion device comprises a cannula, a spring, and a triggering component, wherein the cannula insertion device is configured to insert the cannula into the tissue site of the patient by actuation of the spring released by the triggering component.

3. The glucagon patch pump of claim 1 or 2, wherein the cannula is configured to deploy if the user is in a safe pose when the glucose level drops below the predetermined threshold, and wherein the cannula is configured not to deploy if the user is in an unsafe pose when the glucose level drops below the predetermined threshold.

4. A glucose management system comprising:
the glucagon patch pump of any one of claims 1 to 3;
a glucose sensor;
a processor configured to receive measurements from the glucose sensor and determine a glucose level of the user.

5. The glucose management system of claim 4, comprising an insulin pump.

6. The glucose management system of claim 4, wherein the glucose sensor is a minimally invasive glucose sensor.

7. A method of treating hypoglycemia of a user, the method comprising:
monitoring a user's blood glucose level using a glucose sensor;
detecting when the user's blood glucose level drops below a predetermined threshold;
deploying a cannula into a tissue site of the user in at least in part based on the detection of the blood glucose level dropping below the predetermined threshold; and
administering medication through the cannula from a medication bladder via a medication pump, the medication comprising glucagon.

8. The method of claim 7, comprising issuing a notification to alert the user or a healthcare provider regarding the administration of glucagon or the user's blood glucose level.

9. The method of claim 7 or 8, wherein the deploying comprises actuating a triggering component to allow a pre-loaded spring to drive the cannula into the tissue site.

10. The method of any one of claims 7 to 9, comprising monitoring a pose of the user, wherein the deploying is based at least in part on the user being in a safe pose.

11. The method of any one of claims 7 to 10, wherein the glucose sensor is a minimally invasive glucose sensor.

12. The method of any one of claims 7 to 11, comprising adhering a glucagon patch pump to the user, the glucagon patch pump comprising the cannula, the medication bladder, and the medication pump.

13. The method of any claim 12, comprising connecting the glucagon patch pump to a communication network comprising the glucose sensor.
